# EUROPEAN PATENT APPLICATION

(11) **EP 4 105 215 A1**
(43) Date of publication of application: **21.12.2022**
(21) Application number: 21180320.0
(22) Date of filing: 18.06.2021
(51) Int. Cl.: C07D 471/04, C07C 51/43, C07C 57/15, C07C 65/03, C07C 65/10

(54) **CO-CRYSTAL OF APIXABAN WITH A CARBOXYLIC ACID**

(71) Applicant: University of Limerick, Limerick V94 T9PX (IE)
(72) Inventor: ZAWOROTKO, Michael J., Limerick, V94 T9PX (IE); KUMAR, Vijith, Limerick, V94 T9PX (IE); HASKINS, Molly, Limerick, V94 T9PX (IE)
(74) Representative: Appleyard Lees IP LLP

(57) **Abstract**

cocrystal of formula APX·Bx wherein APX is apixaban (1-(4-methoxyphenyl)-7-oxo-6-(4-(2-oxopiperidin-1-yl)phenyl)-4,5,6,7-tetrahydro-1*H*-pyrazolo[3,4-*c*]pyridine-3-carboxamide), B is a pharmaceutically acceptable carboxylic acid selected from fumaric acid or an aromatic carboxylic acid; and x is from 0.1 to 1.0.

## Description

### Field of the Invention

The present invention relates to a cocrystal comprising apixaban, a method of preparing such a cocrystal, to a pharmaceutical composition comprising such a cocrystal and to a use of such as cocrystal as a medicament. In particular, the present invention relates to a cocrystal comprising apixaban and a pharmaceutically acceptable carboxylic acid selected from fumaric acid or an aromatic carboxylic acid.

### Background of the Invention

Apixaban (1-(4-methoxyphenyl)-7-oxo-6-(4-(2-oxopiperidin-1-yl)phenyl)-4,5,6,7-tetrahydro-1*H-*pyrazolo[3,4-c]pyridine-3- carboxamide; see Formula 1) is hereinafter referred to as APX and is an oral anticoagulant that is a highly potent direct factor Xa inhibitor. It is commonly used against stroke, to treat pulmonary embolism and atrial fibrillation, and to prevent deep vein thrombosis (DVT) for example following hip or knee replacement surgery. APX is commonly used in the anhydrous N-1 form as it is considered to be the thermodynamically stable form under ambient conditions. However APX in this form exhibits low solubility in water (0.028 mg/mL at 24°C) and low oral bioavailability (about 50 % for single 10 mg dose).

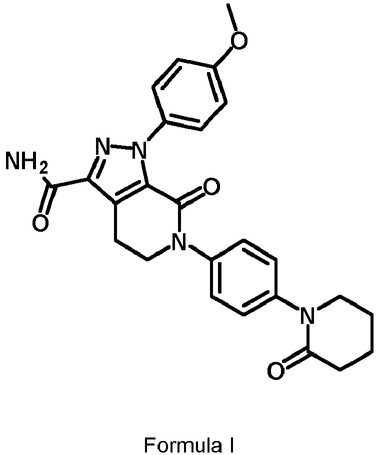

APX has been shown to exhibit various solid forms, including polymorphs, solvates and hydrates. The development of new solid forms of APX with improved physicochemical properties could provide an improved medicine, for example by addressing issues relating to incomplete absorption and first-pass metabolism in the gut and liver. APX is non-ionizable and therefore is believed to be unsuitable for salt formation.

One suitable form of APX is a cocrystal. Common approaches for preparing cocrystals include solution crystallization, slurrying and mechanochemical methods such as grinding. However, solution crystallization can generate large amounts of solvent waste and crystalline defects from grinding can generate partially amorphous materials and/or lead to incomplete conversion to the desired product, thereby requiring further purification step(s).

### Summary of the Invention

According to a first aspect of the present invention, there is provided a cocrystal of formula APX·Bx wherein APX is apixaban (1-(4-methoxyphenyl)-7-oxo-6-(4-(2-oxopiperidin-1-yl)phenyl)-4,5,6,7-tetrahydro-1*H*-pyrazolo[3,4-*c*]pyridine-3- carboxamide), B is a pharmaceutically acceptable carboxylic acid selected from fumaric acid or an aromatic carboxylic acid; and x is from 0.1 to 1.0.

According to a second aspect of the present invention, there is provided a method of preparing a cocrystal according to the first aspect, the method comprising:
(a) admixing apixaban and a pharmaceutically acceptable carboxylic acid selected from fumaric acid or an aromatic carboxylic acid in a solvent; and
(b) stirring the mixture obtained in step (a) at room temperature.

According to a third aspect of the present invention, there is provided a pharmaceutical composition comprising a cocrystal according to the first aspect and a pharmaceutically acceptable excipient.

According to a fourth aspect of the present invention, there is provided a cocrystal according to the first aspect for use as a medicament (for example wherein the medicament is an anticoagulant).

According to a fifth aspect of the present invention, there is provided a cocrystal according to the first aspect for use in the treatment and/or prevention of pulmonary embolism, atrial fibrillation or deep vein thrombosis.

### Detailed Description

It is one aim of the present invention, amongst others, to provide a form of apixaban that addresses at least one disadvantage of the prior art, whether identified here or elsewhere, or to provide an alternative to existing forms. For instance, it may be an aim of the present invention to provide a cocrystal of apixaban which may have improved drug properties compared to apixaban alone and/or other forms of apixaban. It is a further aim of the present invention to provide an improved method of preparing cocrystals comprising apixaban.

According to aspects of the present invention, there is provided a cocrystal, a method of preparing a cocrystal, a pharmaceutical composition comprising a cocrystal and a use of a cocrystal as a medicament, as set forth in the appended claims. Other features of the invention will be apparent from the dependent claims, and the description which follows.

When describing the aspects of the invention, the terms used are to be construed in accordance with the following definitions, unless a context dictates otherwise.

As used in the specification and the appended claims, the singular forms "a", "an," and "the" include both singular and plural referents unless the context clearly dictates otherwise. By way of example, "a solvent" means one solvent or more than one solvent.

As used herein, the term "and/or," when used in a list of two or more items, means that any one of the listed items can be employed by itself or any combination of two or more of the listed items can be employed. For example, if a list is described as comprising group A, B, and/or C, the list can comprise A alone; B alone; C alone; A and B in combination; A and C in combination, B and C in combination; or A, B, and C in combination.

As used herein, unless otherwise expressly specified, all numbers such as those expressing values, ranges, amounts of percentages may be read as if prefaced by the word "about", even if the term does not expressly appear.

The term "about" as used herein when referring to a measurable value such as a parameter, an amount, a temporal duration, and the like, indicate that a value includes the standard deviation of error for the device or method being employed to determine the value. The term "about" is meant to encompass variations of +/-10% or less, +/-5% or less, or +/-0.1 % or less of and from the specified value, insofar such variations are appropriate to perform in the disclosure. It is to be understood that the value to which the modifier "about" refers is itself also specifically disclosed.

The recitation of numerical ranges by endpoints includes all integer numbers and, where appropriate, fractions subsumed within that range (e.g. 1 to 5 can include 1, 2, 3, 4 when referring to, for example, a number of elements, and can also include 1.5, 2, 2.75 and 3.80, when referring to, for example, measurements). The recitation of end points also includes the end point values themselves (e.g. from 1.0 to 5.0 includes both 1.0 and 5.0). Any numerical range recited herein is intended to include all sub-ranges subsumed therein.

As used herein, the term "halogen" or "halo" means fluorine, chlorine, bromine or iodine. Fluorine, chlorine and bromine are particularly preferred.

As used herein, the term "amino" means an NH₂ group.

As used herein, the term "alkoxy" means an alkyl group singularly bonded to oxygen, which is often referred to as "O-R". The term "C₁-C₄ alkoxy" refers to a hydrocarbon (alkyl) chain containing 1, 2, 3 or 4 carbon atoms.

As used herein, the term "alkyl" means both straight and branched chain saturated hydrocarbon groups. Examples of alkyl groups include methyl, ethyl, n-propyl, iso-propyl, n-butyl, t-butyl, i-butyl, and sec-butyl groups.

The term "hydroxy" is used in its usual way, i.e. an oxygen singularly bonded to a hydrogen, "O-H".

As used herein, the term "pharmaceutically acceptable carboxylic acid" means a carboxylic acid that may be used in pharmaceutical compositions, i.e. for administration to a patient to provide treatment. Other acids, which may or may not in themselves be pharmaceutically acceptable, may be useful as intermediates in obtaining the cocrystals of the invention and the pharmaceutically acceptable acids used.

"Therapy", "treatment" and "treating" include both preventative and curative treatment of a condition, disease or disorder. It also includes slowing, interrupting, controlling or stopping the progression of a condition, disease or disorder.

As used herein, the term "room temperature" is used to define a temperature of from about 15 to about 25 °C. The term "room pressure" is used to define atmospheric pressure (1 bar).

As used herein, the term "ambient conditions" is used to define conditions at room temperature and pressure.

Throughout this specification, the term "comprising" or "comprises" means including the component(s) specified but not to the exclusion of the presence of other components. The term "consisting of" or "consists of" means including the components specified but excluding addition of other components. The term "consisting essentially of" or "consists essentially of" means including the components specified but excluding other components except for materials present as impurities, unavoidable materials present as a result of processes used to provide the components, and components added for a purpose other than achieving the technical effect of the invention.

Typically, when referring to compositions, a composition consisting essentially of a set of components will comprise less than 5% by weight, typically less than 3% by weight, more typically less than 1% by weight of non-specified components.

Whenever appropriate, depending upon the context, the use of the term "comprises" or "comprising" may also be taken to encompass or include the meaning "consists essentially of" or "consisting essentially of", and may also be taken to include the meaning "consists of" or "consisting of".

According to a first aspect of the present invention, there is provided a cocrystal of formula APX·Bx wherein APX is apixaban (1-(4-methoxyphenyl)-7-oxo-6-(4-(2-oxopiperidin-1-yl)phenyl)-4,5,6,7 tetrahydro-1H-pyrazolo[3,4-c]pyridine-3-carboxamide), B is a pharmaceutically acceptable carboxylic acid selected from fumaric acid or an aromatic carboxylic acid; and x is from 0.1 to 1.0.

In the cocrystal of the first aspect, the apixaban interacts with the carboxylic acid B to form a cocrystal. This interaction is suitably a non-covalent interaction, suitably a hydrogen bonding interaction. Suitably this interaction at least partially forms and sustains the cocrystal structure.

The carboxylic acid may alternatively or additionally be referred to herein as a coformer.

The cocrystal of the first aspect may be considered to be a crystalline composition comprising the stated cocrystal form, suitably consisting essentially or, or consisting of, the stated cocrystal form. The cocrystal of the first aspect may be additionally or alternatively referred to as a combination active pharmaceutical ingredient, which may be further formulated with suitable pharmaceutically acceptable excipients to provide a pharmaceutical composition for use as a medicament.

The inventors have surprisingly found that apixaban and a pharmaceutically acceptable carboxylic acid selected from fumaric acid or an aromatic carboxylic acid can advantageously form a cocrystal and may provide advantageous properties such as improved solubility, tablet-ability, thermal stability and chemical stability. The cocrystals may advantageously be prepared from a water slurry method, for example at room and/or ambient temperature.

The cocrystal of the first aspect suitably comprises less than 50 wt% of water, for example less than 40 wt% of water or less than 30 wt% of water, relative to the total weight of the cocrystal.

The cocrystal of the first aspect suitably comprises less than 25 wt% of water, for example less than 20 wt% of water, such as less than 15 wt% of water, for example less than 12 wt% of water, relative to the total weight of the cocrystal.

The cocrystal of the first aspect is suitably anhydrous. The term "anhydrous" as used herein means that there are no water molecules of hydration present within the crystal lattice of the cocrystal.

Suitably there may be surface water molecules present (i.e. water molecules present on the exterior surface of the crystal lattice of the cocrystal).

The inventors have surprisingly found that the cocrystal of the first aspect may be prepared in anhydrous form. This is beneficial as hydrous cocrystals usually have poor thermal stability and/or low solubility.

The cocrystal of the first aspect is suitably a crystalline, solid composition.

The cocrystal of the first aspect may be defined by X-ray powder diffraction (XRPD) peaks.

Suitably the cocrystal of the first aspect exhibits distinct X-ray powder diffraction (XPRD) peaks.

Suitably the cocrystal of the first aspect has a X-ray powder diffraction (XRPD) pattern comprising a characteristic peak at an angle 2θ of 7.3°±0.2° as measured by Cu Kα radiation.

Suitably the cocrystal of the first aspect has a X-ray powder diffraction (XRPD) pattern comprising one or more characteristic peaks at an angle 2θ of 5.7°±0.2°, 7.3°±0.2°, 8.8°±0.2°, 11.5°±0.2°, 20.0°±0.2°, 22.0°±0.2° and 23.3°±0.2° as measured by Cu Kα radiation.

Suitably the cocrystal of the first aspect has a X-ray powder diffraction (XRPD) pattern comprising characteristic peaks at an angle 2θ of 5.7°±0.2°, 7.3°±0.2°, 8.8°±0.2°, 11.5°±0.2°, 20.0°±0.2°, 22.0°±0.2° and 23.3°±0.2° as measured by Cu Kα radiation.

Further X-ray powder diffraction (XRPD) peaks may be present. However these are suitably present at a lower intensity than the characteristic peaks at an angle 2θ of 5.7°±0.2°, 7.3°±0.2°, 8.8°±0.2°, 11.5°±0.2°, 20.0°±0.2°, 22.0°±0.2° and 23.3°±0.2° as measured by Cu Kα radiation, when present.

The cocrystal of the first aspect comprises apixaban and a pharmaceutically acceptable carboxylic acid selected from fumaric acid or an aromatic carboxylic acid. Suitably the cocrystal consists essentially of or consists of apixaban and a carboxylic acid selected from fumaric acid or a pharmaceutically acceptable aromatic carboxylic acid.

The cocrystal may comprise apixaban (APX) and fumaric acid (FUM).

When the pharmaceutically acceptable carboxylic acid is an aromatic carboxylic acid, the aromatic carboxylic acid may be a substituted benzoic acid.

For example, the benzoic acid may be substituted with one or more suitable substituents, such as one or more of C₁-C₄ alkoxy, hydroxy, amino, carboxylic acid, ester, amide, halogen, CF₃, CHF₂ or CH₂F groups.

Suitably the aromatic carboxylic acid is a benzoic acid substituted with one or more of C₁-C₄ alkoxy, hydroxy or amino groups.

Suitably the aromatic carboxylic acid is selected from gallic acid (GAL), salicylic acid (SAL), vanillic acid (VAL), 2,3-dihydroxybenzoic acid (2,3HBA), 2,4-dihydroxybenzoic acid (2,4HBA), 2,5-dihydroxybenzoic acid (2,5HBA), 2,6-dihydroxybenzoic acid (2,6HBA), 3,4-dihydroxybenzoic acid (3,4HBA), 3,5-dihydroxybenzoic acid (3,5HBA), 3-hydroxybenzoic acid (3HBA), 4-hydroxybenzoic acid (4HBA) and 4-aminobenzoic acid (4ABA).

Suitably the pharmaceutically acceptable carboxylic acid may be selected from fumaric acid (FUM), gallic acid (GAL), salicylic acid (SAL), vanillic acid (VAL), 2,3-dihydroxybenzoic acid (2,3HBA), 2,4-dihydroxybenzoic acid (2,4HBA), 2,5-dihydroxybenzoic acid (2,5HBA), 2,6-dihydroxybenzoic acid (2,6HBA), 3,4-dihydroxybenzoic acid (3,4HBA), 3,5-dihydroxybenzoic acid (3,5HBA), 3-hydroxybenzoic acid (3HBA), 4-hydroxybenzoic acid (4HBA) and 4-aminobenzoic acid (4ABA).

The cocrystal may comprise apixaban (APX) and gallic acid (GAL).

The cocrystal may comprise apixaban (APX) and salicylic acid (SAL).

The cocrystal may comprise apixaban (APX) and vanillic acid (VAL).

The cocrystal may comprise apixaban (APX) and 2,3-dihydroxybenzoic acid (2,3HBA).

The cocrystal may comprise apixaban (APX) and 2,4-dihydroxybenzoic acid (2,4HBA).

The cocrystal may comprise apixaban (APX) and 2,5-dihydroxybenzoic acid (2,5HBA).

The cocrystal may comprise apixaban (APX) and 2,6-dihydroxybenzoic acid (2,6HBA).

The cocrystal may comprise apixaban (APX) and 3,4-dihydroxybenzoic acid (3,4HBA).

The cocrystal may comprise apixaban (APX) and 3,5-dihydroxybenzoic acid (3,5HBA).

The cocrystal may comprise apixaban (APX) and 3-hydroxybenzoic acid (3HBA).

The cocrystal may comprise apixaban (APX) and 4-hydroxybenzoic acid (4HBA).

The cocrystal may comprise apixaban (APX) and 4-aminobenzoic acid.

x is from 0.1 to 1. Suitably x is less than 0.8, such as less than 0.7, for example less than 0.6.

Suitably x is at least 0.2, for example at least 0.3.

Suitably x is from 0.2 to 0.8, for example from 0.3 to 0.7.

Alternatively or additionally, the cocrystal of the first aspect may be defined by a ratio of apixaban (APX) to carboxylic acid B. For example the cocrystal may comprise APX to B in a ratio of at least 1:1, for example at least 1.2:1 or at least 1.5:1, such as at least 1.8:1 or at least 2:1. Suitably the cocrystal may comprise APX to B in a ratio of from 2:1 or APX to B in a ratio of from 3:1.

In one example, the carboxylic acid B is fumaric acid (FUM) and x is from 0.45 to 0.55, such as 0.5. Suitably the ratio of APX to fumaric acid (FUM) is 2:1.

In one example, the carboxylic acid B is an aromatic carboxylic acid and x is from 0.25 to 0.45, such as 0.25 to 0.4, for example from 0.3 to 0.4. Suitably the ratio of APX to the aromatic carboxylic acid is 3:1.

The cocrystal may comprise apixaban (APX) and fumaric acid (FUM) and x may be from 0.45 to 0.55, such as 0.46. This cocrystal may be referred to herein as APXFUM.

The cocrystal may comprise apixaban (APX) and gallic acid (GAL) and x may be from 0.25 to 0.4, such as 0.31. This cocrystal may be referred to herein as APXGAL.

The cocrystal may comprise apixaban (APX) and salicylic acid (SAL) and x may be from 0.25 to 0.4, such as 0.32. This cocrystal may be referred to herein as APXSAL.

The cocrystal may comprise apixaban (APX) and vanillic acid (VAL). This cocrystal may be referred to herein as APXVAL.

The cocrystal may comprise apixaban (APX) and 2,3-dihydroxybenzoic acid (2,3HBA) and x may be from 0.25 to 0.4, such as 0.33. This cocrystal may be referred to herein as APX2,3HBA.

The cocrystal may comprise apixaban (APX) and 2,4-dihydroxybenzoic acid (2,4HBA) and x may be from 0.25 to 0.4, such as 0.35. This cocrystal may be referred to herein as APX2,4HBA.

The cocrystal may comprise apixaban (APX) and 2,5-dihydroxybenzoic acid (2,5HBA) and x may be from 0.25 to 0.4, such as 0.35. This cocrystal may be referred to herein as APX2,5HBA.

The cocrystal may comprise apixaban (APX) and 2,6-dihydroxybenzoic acid (2,6HBA) and x may be from 0.25 to 0.4, such as 0.32. This cocrystal may be referred to herein as APX2,6HBA.

The cocrystal may comprise apixaban (APX) and 3,4-dihydroxybenzoic acid (3,4HBA) and x may be from 0.25 to 0.4, such as 0.36. This cocrystal may be referred to herein as APX3,4HBA.

The cocrystal may comprise apixaban (APX) and 3,5-dihydroxybenzoic acid (3,5HBA) and x may be from 0.25 to 0.4, such as 0.34. This cocrystal may be referred to herein as APX3,5HBA.

The cocrystal may comprise apixaban (APX) and 3-hydroxybenzoic acid (3HBA) and x may be from 0.25 to 0.4, such as 0.33. This cocrystal may be referred to herein as APX3HBA.

The cocrystal may comprise apixaban (APX) and 4-hydroxybenzoic acid (4HBA) and x may be from 0.25 to 0.4, such as 0.32. This cocrystal may be referred to herein as APX4HBA.

The cocrystal may comprise apixaban (APX) and 4-aminobenzoic acid and x may be from 0.25 to 0.4, such as 0.31. This cocrystal may be referred to herein as APX4ABA.

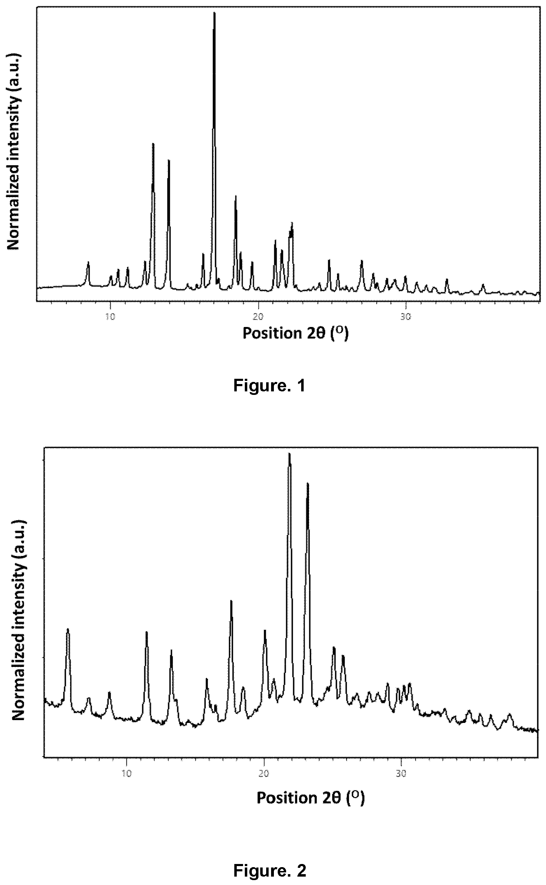

The cocrystal may comprise apixaban and fumaric acid and have an XRPD pattern as shown in Figure 2.

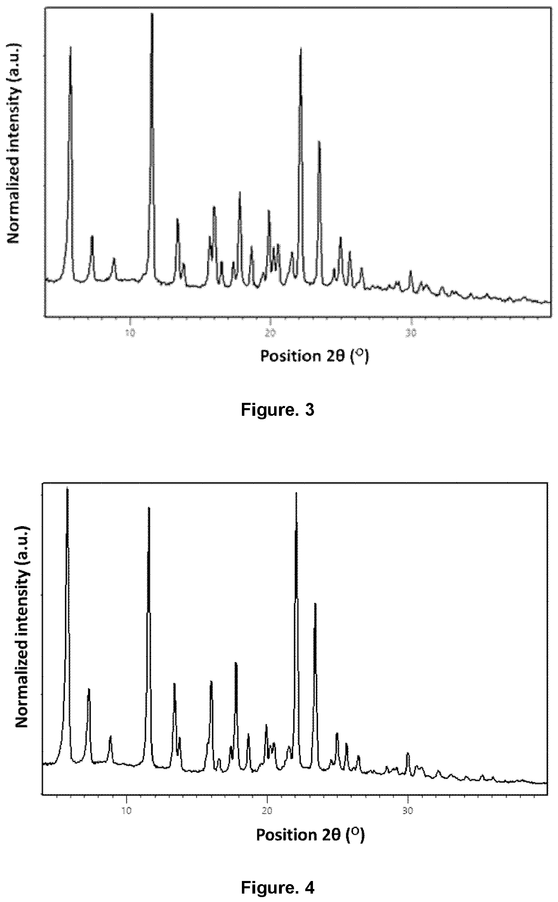

The cocrystal may comprise apixaban and 4-hydroxybenzoic acid and have an XRPD pattern as shown in Figure 3.

The cocrystal may comprise apixaban and 4-aminobenzoic acid and have an XRPD pattern as shown in Figure 4.

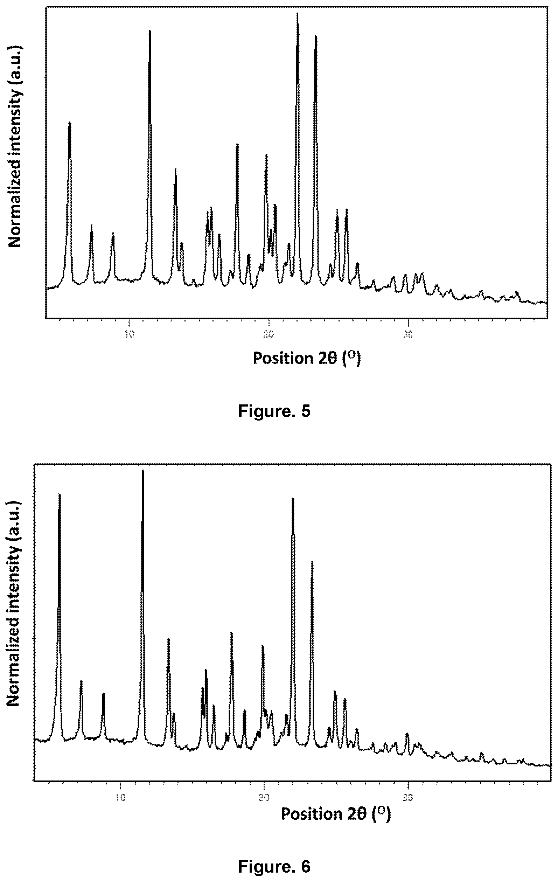

The cocrystal may comprise apixaban and 2,6-dihydroxybenzoic acid and have an XRPD pattern as shown in Figure 5.

The cocrystal may comprise apixaban and 3-hydroxybenzoic acid and have an XRPD pattern as shown in Figure 6.

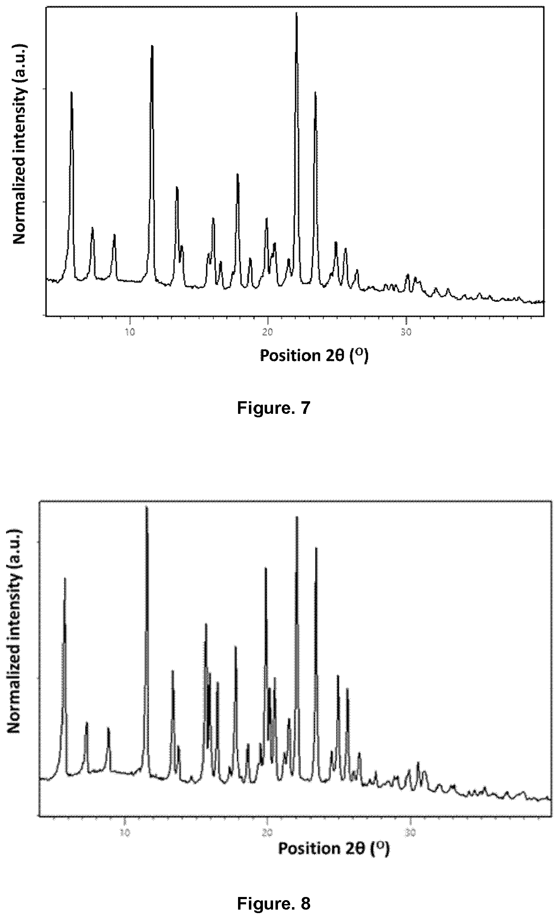

The cocrystal may comprise apixaban and 2,3-dihydroxybenzoic acid and have an XRPD pattern as shown in Figure 7.

The cocrystal may comprise apixaban and 2,5-dihydroxybenzoic acid and have an XRPD pattern as shown in Figure 8.

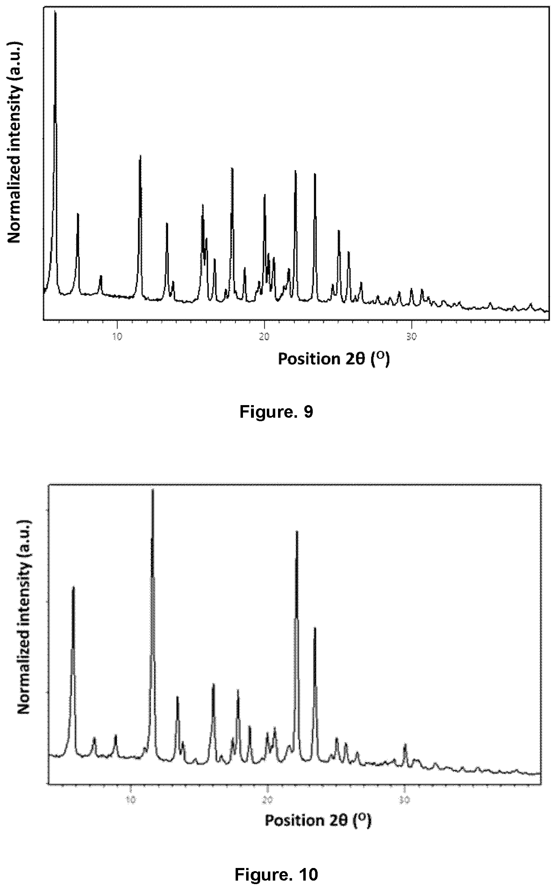

The cocrystal may comprise apixaban and 3,5-dihydroxybenzoic acid and have an XRPD pattern as shown in Figure 9.

The cocrystal may comprise apixaban and 3,4-dihydroxybenzoic acid and have an XRPD pattern as shown in Figure 10.

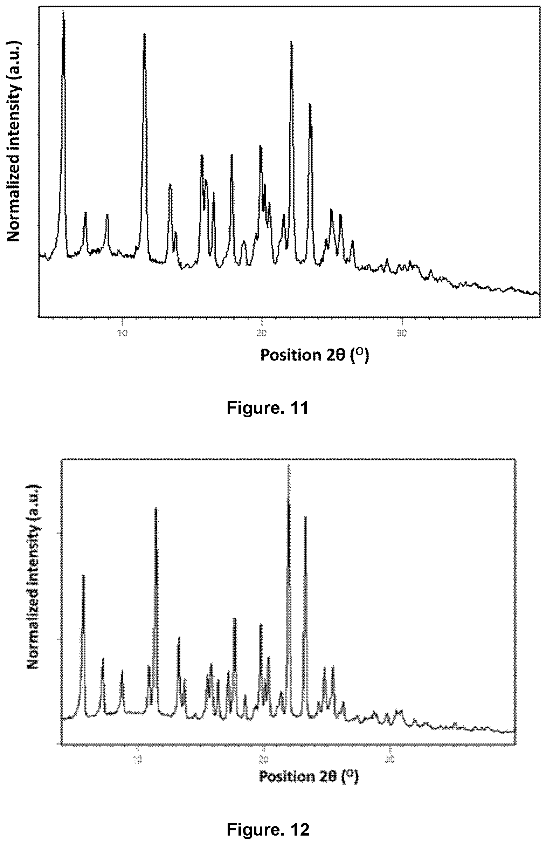

The cocrystal may comprise apixaban and 2,4-dihydroxybenzoic acid and have an XRPD pattern as shown in Figure 11.

The cocrystal may comprise apixaban and salicylic acid and have an XRPD pattern as shown in Figure 12.

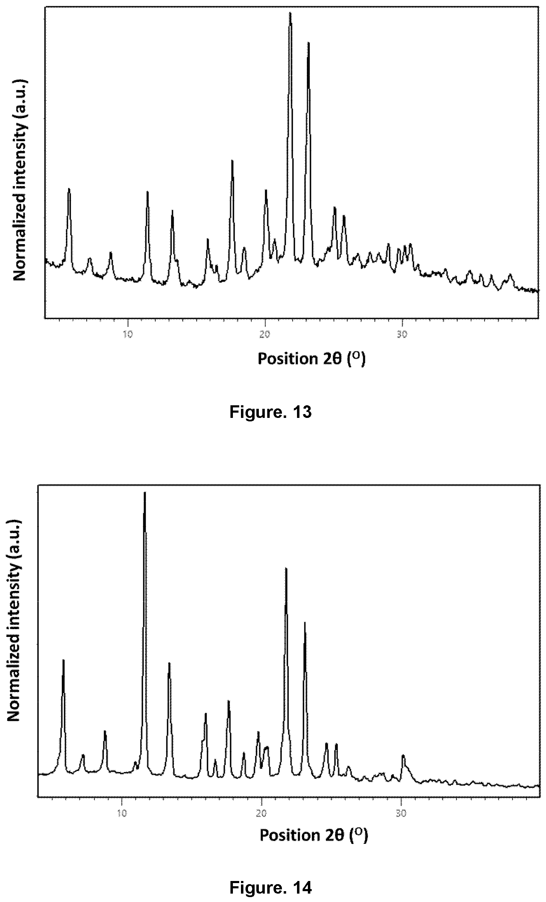

The cocrystal may comprise apixaban and vanillic acid and have an XRPD pattern as shown in Figure 13.

The cocrystal may comprise apixaban and gallic acid and have an XRPD pattern as shown in Figure 14.

The cocrystal of the first aspect may suitably have a melting point of at least 100°C, such as at least 130°C or at least 150°C, for example at least 180°C.

Suitably the cocrystal of the first aspect may have a melting point of less than 300°C, such as less than 270°C or less than 250°C, for example less than 230°C.

Suitably the cocrystal of this first aspect may have a melting point of from 130 to 270°C, for example from 180 to 230°C. Suitable methods for determining the melting point include differential scanning calorimetry (DSC) and will be known to those skilled in the art.

The cocrystal of the first aspect may suitably have a higher solubility in phosphate buffered saline (PBS) than apixaban (N-1 form). The solubility may be measured using high performance liquid chromatography (HPLC).

The cocrystal of the first aspect may suitably have a higher solubility in phosphate buffered saline (PBS) compared to apixaban (N-1 form) at approximately pH 7 and 37°C, for example at pH 6.8 ± 0.2 and 37 °C ± 1°C. Suitably the solubility may be measured in PBS using HPLC, using methods known to the skilled person.

In one example the solubility in PBS may be measured at a time of 60 minutes. This may be referred to herein as "acute" solubility.

In one example the solubility in PBS may be measured at a time of 720 minutes. This may be referred to herein as "chronic" solubility.

The cocrystal may suitably have a solubility in PBS at approximately pH 7 and 37°C which is 5% greater than apixaban (N-1 form), for example 10% or 15% greater, such as 20% or 25% greater, as confirmed by dissolution experiments which will be known to persons skilled in the art.

Suitably the cocrystals of the first aspect are kinetically stable. Suitably the cocrystals of the first aspect do not exhibit a "spring and parachute effect" whereby the cocrystal dissociates into its individual components.

Suitably the cocrystal of the first aspect remain congruent during solubility studies.

The cocrystal of the first aspect may be stable when exposed to a relative humidity of at least 70%. Suitably the cocrystal does not undergo any phase transitions or deform when exposed to a relative humidity of at least 70% as confirmed by thermogravimetric analysis (TGA) and/or XRPD. Thus, the cocrystals of the first aspect have an appropriate stability without the need for special storage measures. Suitably the cocrystal of the first aspect may be stored at room temperature.

The cocrystal of the first aspect may be prepared by methods known in the art. For example, the cocrystal of the first aspect may be prepared using solution crystallization, slurrying or mechanochemical methods such as grinding. However advantageously the cocrystal of the present invention may be prepared in an aqueous solvent such as water.

According to a second aspect of the present invention, there is provided a method of preparing a cocrystal according to the first aspect, the method comprising:
(a) admixing apixaban and a pharmaceutically acceptable carboxylic acid selected from fumaric acid or an aromatic carboxylic acid in a solvent; and
(b) stirring the mixture obtained in step (a) at room temperature.

Suitable solvents for use in preparing the cocrystal of the first aspect include water, acetic acid, acetone, acetonitrile, anisole, 1-butanol, 2-butanol, butyl acetate, tert-butylmethyl ether, chloroform, dimethylformamide, dimethylsulfoxide, 1,4-dioxane, ethanol, ethyl acetate, ethyl ether, ethyl formate, formic acid, heptane, isobutyl acetate, isopropyl acetate, methyl acetate, 3-methyl-1-butanol, methylethyl ketone, methylisobutyl ketone, 2-methyl-1-propanol, pentane, 1-pentanol, methanol, 1-propanol, 2-propanol, propyl acetate, tetrahydrofuran and toluene and mixtures thereof.

The mixture obtained in step (a) may be in the form of a slurry. The cocrystal may be prepared using a slurry method. This method is particularly advantageous as it may produce the most thermodynamically stable form of the cocrystal.

Suitably, according to an aspect of the present invention, there is provided a method of preparing a cocrystal according to the first aspect, the method comprising:
(a) admixing apixaban and a pharmaceutically acceptable carboxylic acid selected from fumaric acid or an aromatic carboxylic acid in a solvent to form a slurry; and
(b) stirring the slurry obtained in step (a) at room temperature.

As will be known by the skilled person, a slurry is a mixture of solids suspended in a solvent. Suitably in the method above the apixaban and pharmaceutically acceptable carboxylic acid are each insoluble or partially soluble in the solvent. Impurities in the apixaban and/or pharmaceutically acceptable carboxylic acid may be soluble in the solvent.

The solvent is suitably an aqueous solvent. Suitably the solvent comprises water. For example, the solvent may comprise water and a miscible liquid. In one aspect, the solvent is water.

In step (b), the mixture obtained in step (a) is stirred at room temperature. Suitably the mixture may be stirred for at least 12 hours, for example at least 24 hours. Suitably the mixture may be stirred for at least 1 day, for example at least 2 days or 3 days. The mixture may suitably be stirred for 4 days.

Preparing the cocrystal in a solvent (particularly a solvent comprising water) is a facile, low cost method for synthesising pure cocrystals in high yield. It is also suitable for large scale manufacturing.

Water is a highly desirable solvent for industrial use as it is non-toxic, eco-friendly, inexpensive and readily available. However water is not typically suited as a solvent for such methods as it may afford hydrates or cause the cocrystal to dissociate into its constituents. The inventors have surprisingly found that cocrystals (particularly the anhydrous cocrystals) of the present invention may be prepared using water as a solvent.

Suitably, according to an aspect of the present invention, there is provided a method of preparing a cocrystal according to the first aspect, the method comprising:
(a) admixing apixaban and a pharmaceutically acceptable carboxylic acid selected from fumaric acid or an aromatic carboxylic acid in water to form a slurry; and
(b) stirring the slurry obtained in step (a) at room temperature.

The method of the second and related aspects are suitably performed at room temperature and pressure. This is advantageous when compared to methods of the prior art which typically make use of elevated temperatures.

The method of the second aspect suitably further comprises:
(c) removing the solvent from the mixture (for example slurry) obtained in step (b), for example by drying the mixture obtained in step (b), to obtain the cocrystal (for example in the form of a solid powder).

Suitably the mixture obtained in step (b) may be filtered, for example filtered under vacuum. For example, the mixture obtained in step (b) may be filtered prior to removal of the solvent, for example by drying.

Any suitable method of drying may be used. Suitable methods may include drying in an oven at a temperature of about 60 to 80 °C.

Further purification steps may be performed as necessary. However suitably no further purification steps are necessary. This is a significant advantage over methods of the prior art requiring purification steps.

According to an aspect of the present invention, there is provided a method of preparing a cocrystal of formula APX·Bx wherein APX is apixaban (1-(4-methoxyphenyl)-7-oxo-6-(4-(2-oxopiperidin-1-yl)phenyl)-4,5,6,7-tetrahydro-1*H*-pyrazolo[3,4-*c*]pyridine-3- carboxamide), B is a pharmaceutically acceptable carboxylic acid selected from fumaric acid and an aromatic carboxylic acid; and x is 0.1 to 1.0; the method the method comprising the steps of:
(a) admixing apixaban and a pharmaceutically acceptable carboxylic acid selected from fumaric acid or an aromatic carboxylic acid in an aqueous solvent; and
(b) stirring the mixture obtained in step (a) at room temperature.

Preferred features of this aspect are as described as in relation to the first and second aspects.

According to a third aspect of the present invention, there is provided a pharmaceutical composition comprising a cocrystal of the first aspect and a pharmaceutically acceptable excipient.

Pharmaceutically acceptable excipients include fillers, binders, lubricants, flavours, preservatives, colourings, disintegrants, suspending agents, stabilizing agents and coatings. Examples of such components are known to the person skilled in the art.

According to a fourth aspect of the present invention, there is provided a cocrystal of the first aspect for use as a medicament. For example, the medicament may be an anticoagulant.

According to a fifth aspect of the present invention, there is provided a cocrystal of the first aspect for use in the treatment and/or prevention of pulmonary embolism, atrial fibrillation or deep vein thrombosis.

According to a sixth aspect of the present invention, there is provided a method of treating and/or preventing pulmonary embolism, atrial fibrillation or deep vein thrombosis, the method comprising administering to a patient a cocrystal of the first aspect.

According to a seventh aspect of the present invention, there is provided a pharmaceutical composition of the third aspect for use in the treatment and/or prevention of pulmonary embolism, atrial fibrillation or deep vein thrombosis.

According to an eighth aspect of the present invention, there is provided a method of treating and/or preventing pulmonary embolism, atrial fibrillation or deep vein thrombosis, the method comprising administering to a patient a pharmaceutical composition of the third aspect.

According to a ninth aspect of the present invention, there is provided a cocrystal obtained or obtainable by the method of preparing a cocrystal according to the second aspect.

Preferred features of the third, fourth, fifth, sixth, seventh, eighth and ninth aspects may have any of the suitable features and advantages described in relation to the first aspect.

### Brief Description of the Figures

Figure 1: XRPD pattern of APX N-1 form.
Figure 2: XRPD pattern of anhydrous cocrystal APXFUM.
Figure 3: XRPD pattern of anhydrous cocrystal APX4HBA.
Figure 4: XRPD pattern of anhydrous cocrystal APX4ABA.
Figure 5: XRPD pattern of anhydrous cocrystal APX2,6HBA.
Figure 6: XRPD pattern of anhydrous cocrystal APX3HBA.
Figure 7: XRPD pattern of anhydrous cocrystal APX2,3HBA.
Figure 8: XRPD pattern of anhydrous cocrystal APX2,5HBA.
Figure 9: XRPD pattern of anhydrous cocrystal APX3,5HBA.
Figure 10: XRPD pattern of anhydrous cocrystal APX3,4HBA.
Figure 11: XRPD pattern of anhydrous cocrystal APX2,4HBA.
Figure 12: XRPD pattern of anhydrous cocrystal APXSAL.
Figure 13: XRPD pattern of anhydrous cocrystal APXVAL.
Figure 14: XRPD pattern of anhydrous cocrystal APXGAL.
Figure 15: Comparison of calculated XRPD pattern of cocrystal APXFUM with experimental XRPD pattern of APXFUM, APX4HBA, APX3HBA, APX4ABA, APX2,6HBA, APX2,3HBA, APX2,5HBA.
Figure 16: Comparison of calculated XRPD pattern of cocrystal APXFUM with experimental XRPD pattern of APXFUM, APX3,5HBA, APX3,4HBA, APX2,4HBA, APXSAL, APXGAL, APXVAL.

For a better understanding of the invention, and to show how embodiments of the same may be carried into effect, reference will now be made, by way of example, to the following experimental data.

### Detailed Description Of The Example Embodiments

Thirteen new APX cocrystals were obtained by a water slurry method. APX (Ambeed Inc. 98 % purity) and coformers (Sigma Aldrich) were purchased commercially and used without any further purification. Deionised water was used for the synthesis of the cocrystals.

### Example 1: Cocrystal of apixaban (APX) and fumaric acid (FUM) - APXFUM

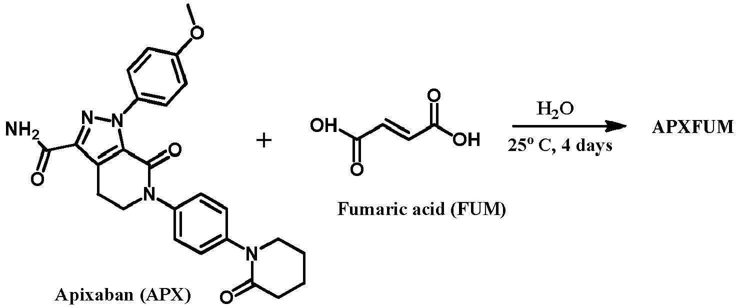

APX (500 mg, 1.09 mmol) and FUM (64 mg, 0.55 mmol, as shown in Formula II) were mixed in a glass vial, 10 mL of water was added and the resulting slurry was stirred at 25°C for 4 days. Solvent was removed by filtering under vacuum and the obtained powder was dried in an oven (70°C for 2 hours) to obtain a white powder, Yield 96 %, m.p. 228°C.

### Example 2: Cocrystal of apixaban (APX) and 4-hydroxybenzoic acid (4HBA) - APX4HBA

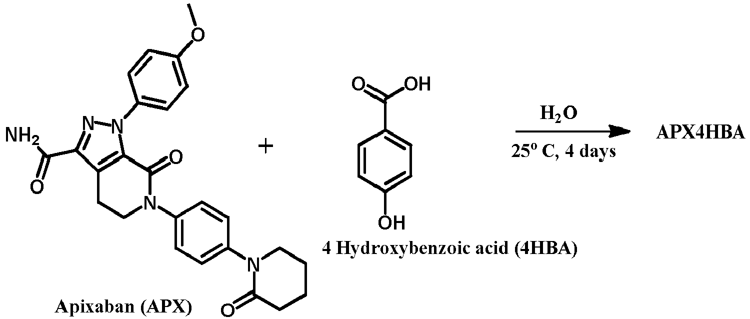

APX (500 mg, 1.09 mmol) and 4HBA (50 mg, 0.36 mmol) were mixed in a glass vial, 10 mL of water was added and the resulting slurry was stirred at 25°C for 4 days. Solvent was removed by filtering under vacuum and the obtained powder was dried in an oven (70°C for 2 hours) to obtain a white powder, Yield 97 %, m.p. 200°C.

### Example 3: Cocrystal of apixaban (APX) and 4-aminobenzoic acid (4ABA) - APX4ABA

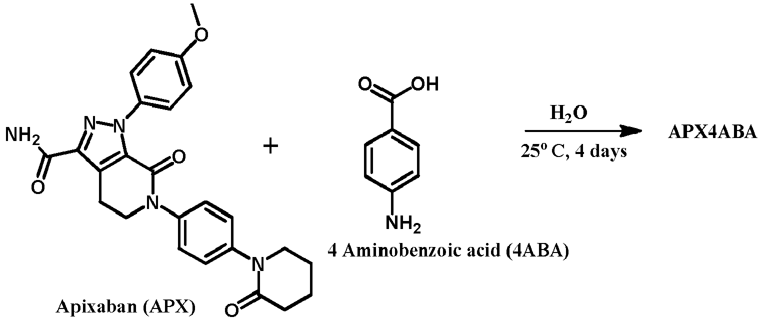

APX (500 mg, 1.09 mmol) and 4ABA (50 mg, 0.36 mmol) were mixed in a glass vial, 10 mL of water was added and the resulting slurry was stirred at 25°C for 4 days. Solvent was removed by filtering under vacuum and the obtained powder was dried in an oven (70°C for 2 hours) to obtain a white powder, Yield 95 %, m.p. 198°C

### Example 4: Cocrystal of apixaban (APX) and 2,6-dihydroxybenzoic acid (2,6HBA) - APX2,6HBA

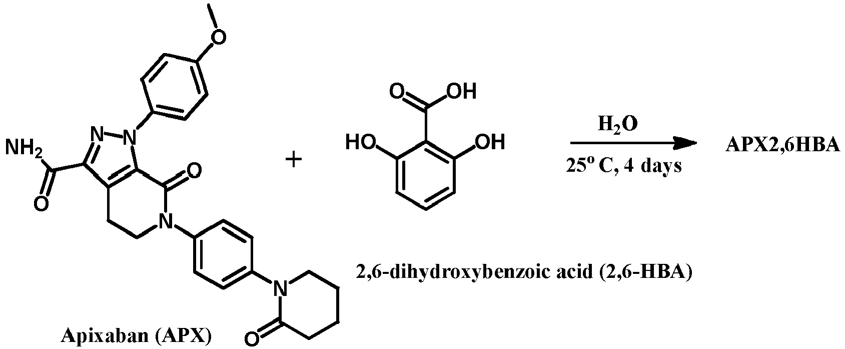

APX (500 mg, 1.09 mmol) and 2,6HBA (56 mg, 0.36 mmol) were mixed in a glass vial, 10 mL of water was added and the resulting slurry was stirred at 25°C for 4 days. Solvent was removed by filtering under vacuum and the obtained powder was dried in an oven (70°C for 2 hours) to obtain a white powder, Yield 95 %, m.p. 189°C.

### Example 5: Cocrystal of apixaban (APX) and 3-hydroxybenzoic acid (3HBA) - APX3HBA

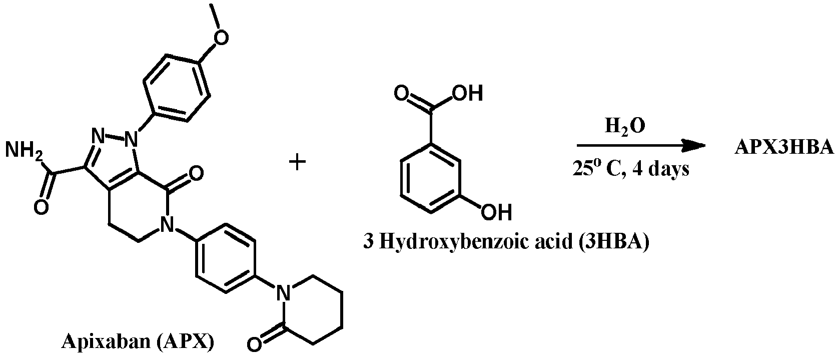

APX (500 mg, 1.09 mmol) and 3HBA (50 mg, 0.36 mmol) were mixed in a glass vial, 10 mL of water was added and the resulting slurry was stirred at 25°C for 4 days. Solvent was removed by filtering under vacuum and the obtained powder was dried in an oven (70°C for 2 hours) to obtain a white powder, Yield 96 %, m.p. 215°C.

### Example 6: Cocrystal of apixaban (APX) and 2,3-dihydroxybenzoic acid (2,3HBA) - APX2,3HBA

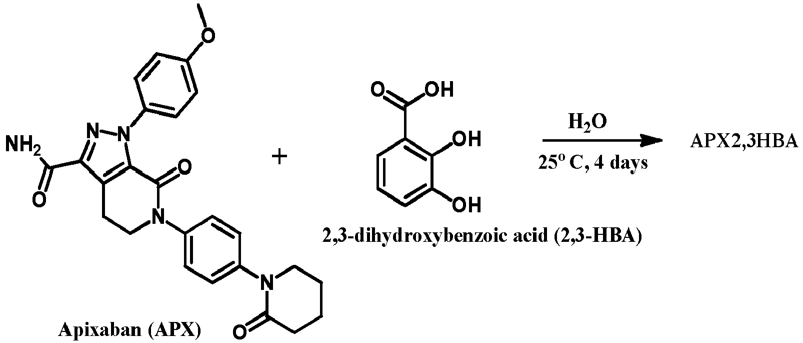

APX (500 mg, 1.09 mmol) and 2,3HBA (56 mg, 0.36 mmol) were mixed in a glass vial, 10 mL of water was added and the resulting slurry was stirred at 25°C for 4 days. Solvent was removed by filtering under vacuum and the obtained powder was dried in an oven (70°C for 2 hours) to obtain a white powder, Yield 94 %, m.p. 195°C.

### Example 7: Cocrystal of apixaban (APX) and 2,5-dihydroxybenzoic acid (2,5HBA) - APX2,5HBA

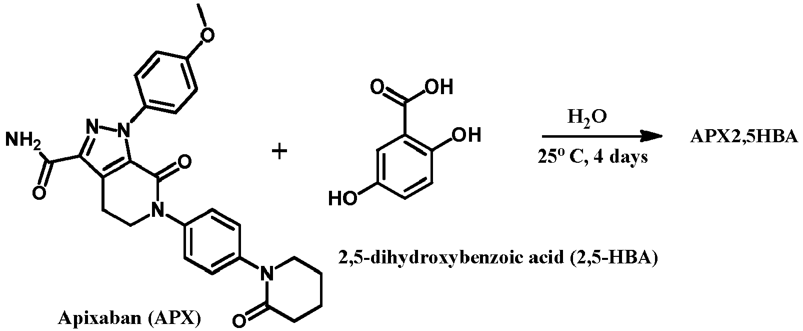

APX (500 mg, 1.09 mmol) and 2,5HBA (56 mg, 0.36 mmol) were mixed in a glass vial, 10 mL of water was added and the resulting slurry was stirred at 25°C for 4 days. Solvent was removed by filtering under vacuum and the obtained powder was dried in an oven (70°C for 2 hours) to obtain a white powder, Yield 96 %, m.p. 211°C.

### Example 8: Cocrystal of apixaban (APX) and 3,5-dihydroxybenzoic acid (3,5HBA) - APX3,5HBA

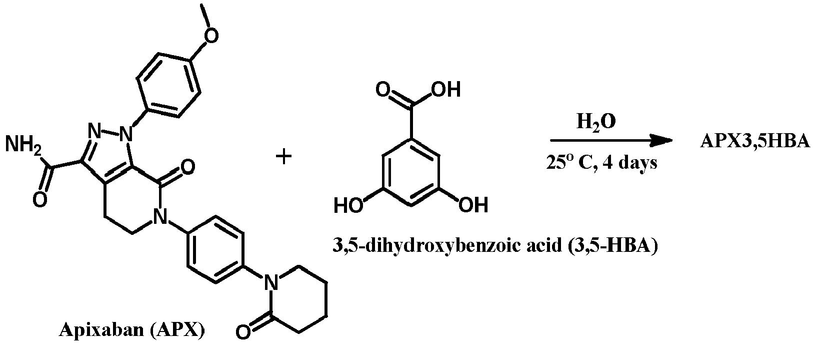

APX (500 mg, 1.09 mmol) and 3,5HBA (56 mg, 0.36 mmol) were mixed in a glass vial, 10 mL of water was added and the resulting slurry was stirred at 25°C for 4 days. Solvent was removed by filtering under vacuum and the obtained powder was dried in an oven (70°C for 2 hours) to obtain a white powder, Yield 96 %, m.p. 216°C.

### Example 9: Cocrystal of apixaban (APX) and 3,4-dihydroxybenzoic acid (3,4HBA) - APX3,4HBA

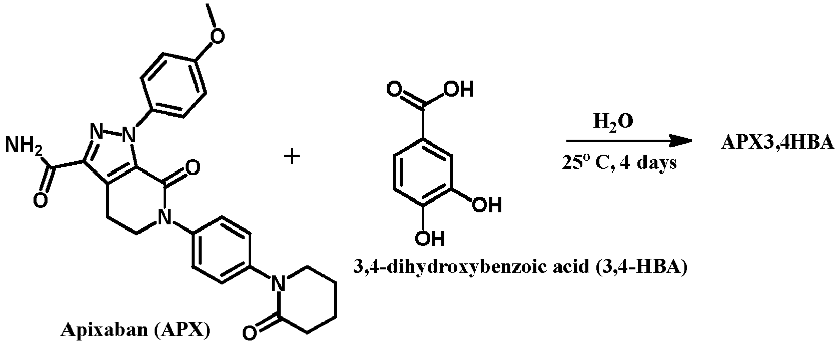

APX (500 mg, 1.09 mmol) and 3,4HBA (56 mg, 0.36 mmol) were mixed in a glass vial, 10 mL of water was added and the resulting slurry was stirred at 25°C for 4 days. Solvent was removed by filtering under vacuum and the obtained powder was dried in an oven (70°C for 2 hours) to obtain a white powder, Yield 96 %, m.p. 210°C.

### Example 10: Cocrystal of apixaban (APX) and 2,4-dihydroxybenzoic acid (2,4HBA) - APX2,4HBA

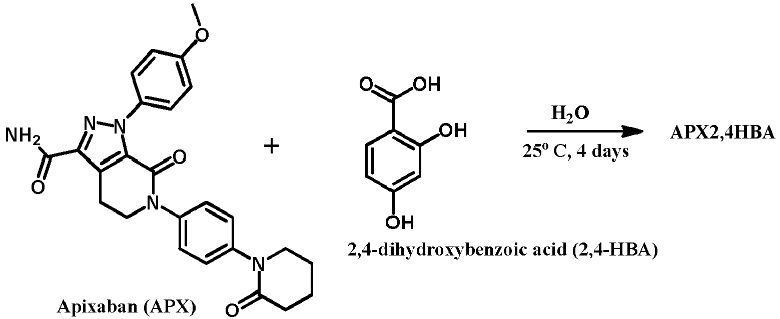

APX (500 mg, 1.09 mmol) and 2,4HBA (56 mg, 0.36 mmol) were mixed in a glass vial, 10 mL of water was added and the resulting slurry was stirred at 25°C for 4 days. Solvent was removed by filtering under vacuum and the obtained powder was dried in an oven (70°C for 2 hours) to obtain a white powder, Yield 95 %, m.p. 204°C.

### Example 11: Cocrystal of apixaban (APX) and salicylic acid (SAL) - APXSAL

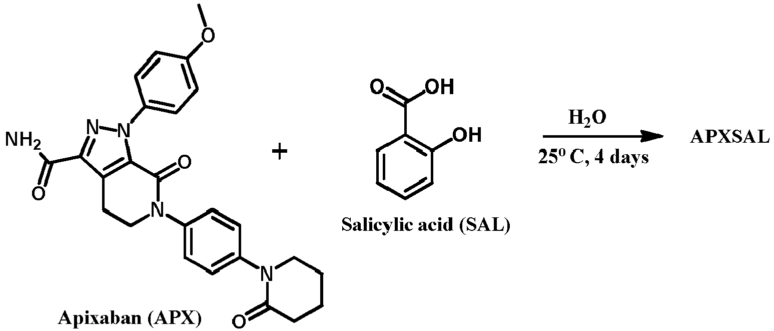

APX (500 mg, 1.09 mmol) and SAL (50 mg, 0.36 mmol) were mixed in a glass vial, 10 mL of water was added and the resulting slurry was stirred at 25°C for 4 days. Solvent was removed by filtering under vacuum and the obtained powder was dried in an oven (70°C for 2 hours) to obtain a white powder, Yield 93 %, m.p. 193°C.

### Example 12: Cocrystal of Apixaban (APX) and vanillic acid (VAL) - APXVAL

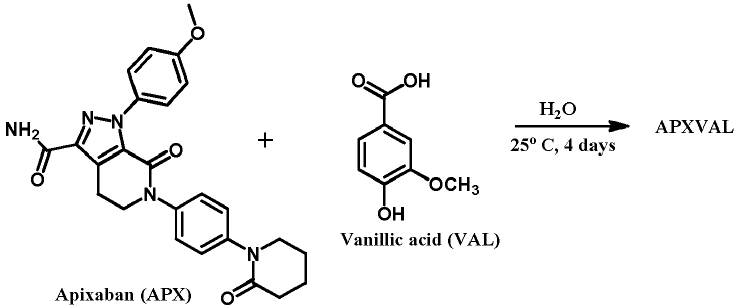

APX (500 mg, 1.09 mmol) and VAL (61 mg, 0.36 mmol) were mixed in a glass vial, 10 mL of water was added and the resulting slurry was stirred at 25°C for 4 days. Solvent was removed by filtering under vacuum and the obtained powder was dried in an oven (70 °C for 2 hours) to obtain a white powder, Yield 93 %, m.p. 195°C.

### Example 13: Cocrystal of apixaban (APX) and gallic acid (GAL) - APXGAL

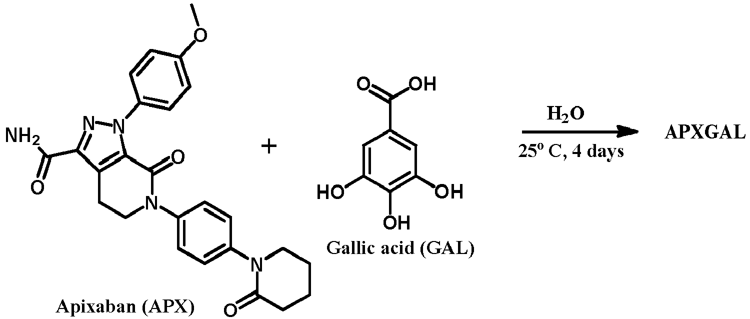

APX (500 mg, 1.09 mmol) and GAL (62 mg, 0.36 mmol) were mixed in a glass vial, 10 mL of water was added and the resulting slurry was stirred at 25°C for 4 days. Solvent was removed by filtering under vacuum and the obtained powder was dried in an oven (70 °C for 2 hours) to obtain a white powder, Yield 95 %, m.p. 212°C.

### Example 14: Apixaban (APX) - N-1 form

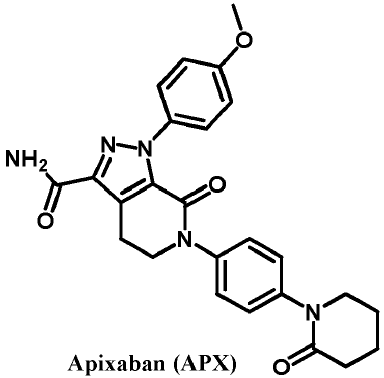

APX (Ambeed Inc. 98 % purity) was purchased commercially and used without any further purification.

The cocrystals of Examples 1 to 13 and apixaban in N-1 form (Example 14) were characterised by X-ray powder diffraction (XRPD), thermogravimetric analysis (TGA), differential scanning calorimetry (DSC), 1H solution NMR spectroscopy and high performance liquid chromatography (HPLC), as follows: .

### 1) X-ray Powder Diffraction (XRPD)

Diffractograms were recorded using a PANalytical Empyrean^{™} diffractometer equipped with a PIXcel3D detector operating in scanning line detector mode with an active length of 4 utilizing 255 channels. The diffractometer is outfitted with an Empyrean Cu LFF (long fine-focus) HR (9430 033 7310x) tube operated at 40 kV and 40 mA and Cu Kα radiation (λα = 1.540598 Å) was used for diffraction experiments. Continuous scanning mode with the goniometer in the 2 theta-theta orientation was used to collect the data. Incident beam optics included the Fixed Divergences slit with anti-scatter slit PreFIX module, with a 1/8° divergence slit and a 1/4° anti-scatter slit, as well as a 10 mm fixed incident beam mask and a Soller slit (0.04 rad). Divergent beam optics included a P7.5 anti-scatter slit, a Soller slit (0.04 rad), and a Ni-β filter. In a typical experiment, approximately 20 mg of sample was dried, ground into a fine powder and was loaded on a zero background silicon discs. The data were collected from 5°- 40° (2θ) with a step-size of 0.02626° and a scan time of 29 seconds per step. Raw data was analyzed using the X'Pert HighScore Plus^{™} software V 4.1 (PANalytical, The Netherlands).

### 2) Differential Scanning Calorimetry (DSC)

DSC tests were performed using a TA Q2000 heat flux Differential Scanning Calorimeter. Purge gas used was dry nitrogen, the reference material was an empty aluminium crimped pan and the sample purge was 50 mL/minute. The starting temperature was typically 20°C with a heating rate of 10°C/ minute, and the ending temperature was set to 300°C for all experiments. The data were evaluated using T.A. Universal Analysis suite for Windows XP/Vista Version 4.5A. Typically, the first thermal event was attributed to melting of the cocrystal.

### 3) Thermogravimetric analysis (TGA)

Thermograms were recorded under nitrogen using a TA Q50 V20.13 Build 39 instrument. Platinum pans and a flow rate of 60 mL/min for the nitrogen gas were used for the experiments. The data were collected in the High Resolution Dynamic mode with a sensitivity of 1.0, a resolution of 4.0, and a temperature ramp of 20°C/min up to 500°C. The data were evaluated using T.A. Universal Analysis suite for Windows XP/Vista Version 4.5A. The TGA results were used to evaluate the thermal stability (see Table 2) and stoichiometry (see Table 3) of the cocrystals.

### 4) Nuclear Magnetic Resonance Spectroscopy (NMR)

¹H NMR spectra were recorded at ambient temperature on a Joel EX270 spectrometer with *B₀* = 7.05 T. Data were collected at ambient temperature and DMSO-*d*₆, was used as solvent. NMR spectra were analysed using Mestrelab Mnova software package. The NMR spectra provided the stoichiometry of each cocrystals based upon the integration of relevant peaks (see Table 3).

### 5) High Performance Liquid Chromatography (HPLC)

25 mg equivalent of APX was sieved to 75-100µm using standard-mesh sieve and the resulting slurry was added to 500 mL phosphate buffer solution (PBS) 6.8 at 37°C stirring at 100 rpm. 1mL aliquots were taken at 1 3, 5, 10, 15, 30, 60, 120, 240, 480, 720 and 1440 minutes. Each aliquot filtered through a 0.45 µm Whattmann syringe filter. 500 µL of filtered aliquot and 500 µL of methanol were pipetted into a vial and injected to HPLC. All dissolution experiments for APX and cocrystals were carried in triplicates.

The dissolution aliquots were analysed using a Shimadzu (LC-20A) HPLC instrument with a Gemini^{®} C18 (250 x 4.6 x 5 µm) column. The wavelength was set to 235nm with an injection volume of 10 µL and a flow rate of 1 mL/min with the oven set at 40°C. The mobile phase was comprised of 25 %: 75 % (0.1 % orthophosphoric acid in acetonitrile:water) with a gradient to 100 % from 0-13mins (0.1 % orthophosphoric acid in acetonitrile) held at 100 % to 15.5 minutes and reduced to 25 %:75 % (0.1 % orthophosphoric acid in acetonitrile: water) over 2 minute gradient and left at this composition for a further 4 minutes.

X-ray powder diffraction (XRPD) was used to identify the new crystalline solid forms. The XRPD patterns for APX and Examples 1 to 13 are provided in Figures 1 to 14.

The XRPD patterns indicate isostructurality of the 13 cocrystals. Each of Examples 1 to 13 can be identified by 7 characteristic peaks (see Table 1). These characteristic peaks are distinct from those of the starting materials.

**Table 1: Characteristic XRPD peaks of APX, carboxylic acids/coformers and APX cocrystals measured using Cu radiation.**

| **Individual components** | **Characteristic peaks (2θ) (± 0.2 degrees)** | **Cocrystal** | **Characteristic peaks (2θ) (± 0.2 degrees)** |
|---|---|---|---|
| APX | 12.9, 13.9, 17.0, 21.1, 22.2 | APXFUM | 5.7, 7.3, 8.8, 11.5, 20.1, 21.9, 23.3 |
| FUM | 18.6, 22.8, 24.7, 28.8, 29.4 | | |
| 4HBA | 17.5, 19.4, 24.4, 26.8, 29.9 | APX4HBA | 5.7, 7.3, 8.8, 11.5, 19.9, 22.1, 23.4 |
| 4ABA | 9.5, 13.9, 15.4, 21.9, 35.9 | APX4ABA | 5.8, 7.2, 8.8, 11.6, 20.1, 22.1, 23.4 |
| 2,6HBA | 12.0, 14.5, 24.4, 25.2, 27.4 | APX2,6HBA | 5.7, 7.3, 8.8, 11.5, 19.9, 22.0, 23.3 |
| 3HBA | 8.8, 16.7, 17.6, 23.7, 26.6 | APX3HBA | 5.7, 7.3, 8.8, 11.5, 19.9, 21.9, 23.3 |
| 2,3HBA | 10.7, 12.6, 13.4, 16.4, 27.8 | AX2,3HBA | 5.8, 7.3 8.9, 11.6, 19.9, 22.0, 23.4 |
| 2,5HBA | 7.6, 16.1, 19.9, 25.3, 27.0 | APX2,5HBA | 5.7, 7.3, 8.8, 11.5, 19.9, 22.1, 23.4 |
| 3,5HBA | 7.6, 16.0, 19.8, 22.6, 26.9 | APX3,5HBA | 5.7, 7.3, 8.8, 11.5, 19.9, 22.1, 23.4 |
| 3,4HBA | 15.5, 16.9, 20.8, 25.5, 26.9 | APX3,4HBA | 5.8, 7.3, 8.9, 11.6, 20.0, 22.1, 23.4 |
| 2,4HBA | 13.8, 16.4, 25.5, 27.0, 28.3 | APX2,4HBA | 5.8, 7.3, 8.9, 11.6, 19.9, 22.1, 23.4 |
| SAL | 10.9, 17.2, 25.5, 28.1, 28.7 | APXSAL | 5.7, 7.2, 8.8, 11.5, 19.9, 22.0, 23.3 |
| VAL | 9.5, 10.3, 12.9, 16.6, 27.1 | APXVAL | 5.7, 7.3, 8.8, 11.5, 20.0, 22.0, 23.4 |
| GAL | 8.1, 11.9, 16.2, 19.1, 32.6 | APXGAL | 5.8, 7.2, 8.8, 11.6, 19.9, 21.9, 23.2 |

The stoichiometry of APX to coformers in the cocrystals was determined by solution 1H NMR spectroscopy and revealed that the weight % coformers is less than 12 % for each coformer.

The thermal properties of the individual coformers and cocrystals of Examples 1 to 13 are provided in Table 2.

**Table 2: Thermal properties of APX, coformers and APX cocrystals**

| **DSC data** | | | | **TGA data** |
|---|---|---|---|---|
| **Individual components** | **Melting point (°C)** | **Cocrystal** | **Melting point (°C)** | **Experimental Weight loss (%)** |
| APX | 238 | APXFUM | 228 | 11.28 |
| FUM | 298 | | | |
| 4HBA | 214 | APX4HBA | 200 | 9.05 |
| 4ABA | 187 | APX4ABA | 198 | 8.2 |
| 2,6HBA | 165 | APX2,6HBA | 189 | 9.71 |
| 3HBA | 202 | APX3HBA | 215 | 9.72 |
| 2,3HBA | 204 | APX2,3HBA | 195 | 9.64 |
| 2,5HBA | 200 | APX2,5HBA | 211 | 10.23 |
| 3,5HBA | 236 | APX3,5HBA | 216 | 10.48 |
| 3,4HBA | 197 | APX3,4HBA | 210 | 10.05 |
| 2,4HBA | 208 | APX2,4HBA | 204 | 9.12 |
| SAL | 158 | APXSAL | 193 | 8.90 |
| VAL | 353 | APXVAL | 195 | 9.83 |
| GAL | 260 | APXGAL | 212 | 10.91 |

DSC and TGA analysis confirmed that the cocrystals are stable to at least 190°C and are anhydrous

The stoichiometry of APX to coformers in the cocrystals was determined by solution 1H NMR spectroscopy (see Table 3).

**Table 3: Solution ¹H NMR spectroscopy data for cocrystals, illustrating the stoichiometry of each cocrystal.**

| **Cocrystal** | **1HNMR δ, integration value, number of proton, molar ratio** | **Molar ratio of the coformer: APX (1)** |
|---|---|---|
| APXFUM | 6.99 0.94 (2H) 0.46 | 0.46 |
| APXGAL | 6.92 0.62 (2H) 0.31 | 0.31 |
| APXVAL | n/a | n/a |
| APXSAL | 7.80, 0.34 (1 H) 0.34 | 0.32 |
| | 6.94, 0.61 (2H) 0.31 | |
| APX3HBA | 7.33, 0.69 (2H) 0.34 | 0.33 |
| | 7.28, 0.33 (1H) 0.33 | |
| | 7.01, 0.36 (1H) 0.33 | |
| APX4HBA | 7.79 , 0.66 (2H) 0.33 | 0.32 |
| | 6.82, 0.64 (2H) 0.32 | |
| APX4ABA | 7.60, 0.62 (2H) 0.31 | 0.31 |
| | 6.54, .0.62 (2H) 0.31 | |
| APX2,3HBA | 7.23, 0.34 (1H) 0.34 | 0.33 |
| | 7.01, 0.32 (1H) 0.32 | |
| | 6.70, 0.34 (1H) 0.34 | |
| APX2,4HBA | 7.62, 0.35 (1H) 0.35 | 0.35 |
| | 6.34, 0.35 (1H) 0.35 | |
| | 6.25, 0.34 (1H) 0.34 | |
| APX2,5HBA | 7.12, 0.35 (1H) 0.35 | 0.35 |
| | 6.91, 0.36 (1H) 0.36 | |
| | 6.76, 0.35 (1H) 0.35 | |
| APX2,6HBA | 6.27, 0.95 (3H) 0.32 | 0.32 |
| APX3,4HBA | 7.33, 0.84 (2H) (B) | 0.36 |
| | 6.77, 0.36 (1H) 0.36 | |
| APX3,5HBA | 7.15, 0.33 (1H) 0.33 | 0.34 |
| | 6.94, 0.36 (1H) 0.36 | |
| | 6.79, 0.34 (1H) 0.34 | |

Physicochemical properties such as PBS 6.8 solubility were analysed by high performance liquid chromatography (HPLC). Accelerated stability tests were conducted on each cocrystal and the acute and chronic solubility of the APX N-1 form and the cocrystals of Examples 1 to 13 are presented in Table 4.

**Table 4: Solubility of APX and respective cocrystals in PBS 6.8 at 37°C**

| **Cocrystal** | **Acute, T= 60 mins Conc (mg/mL)** | **Chronic, T= 720mins Conc (mg/mL)** |
|---|---|---|
| APX | 0.0241 | 0.0303 |
| APXSAL | 0.0285 | 0.0442 |
| APXFUM | 0.0251 | 0.0422 |
| APXGAL | 0.0272 | 0.0466 |
| APXVAL | 0.0321 | 0.0466 |
| APX4ABA | 0.0288 | 0.0480 |
| APX3HBA | 0.0255 | 0.0465 |
| APX4HBA | 0.0293 | 0.0453 |
| APX2,3HBA | 0.0214 | 0.0479 |
| APX2,4HBA | 0.0292 | 0.0458 |
| APX2,5HBA | 0.0312 | 0.0455 |
| APX2,6HBA | 0.0315 | 0.0455 |
| APX3,4HBA | 0.0220 | 0.0429 |
| APX3,5HBA | 0.0292 | 0.0435 |

Stability of the cocrystals were studied based on the USFDA protocol of Guidance for Industry Q1A(R2) Stability Testing of New Drug Substances and Products. Two weeks accelerated stability studies of the cocrystals were performed at 40°C ± 2°C/75% RH ± 5%. TGA and XRPD analysis reveals all cocrystals are stable, anhydrous and exhibit no phase changes with the presence of humidity.

The cocrystals of Examples 1 to 13 each exhibited an increase in PBS solubility compared to the N-1 form of APX (example 14) as analysed using HPLC in triplicate.

The kinetic stability of the cocrystals after PBS 6.8 dissolution experiments was supported by the lack of a "spring-and-parachute" effect and XRPD studies of the recovered solids. Accelerated stability tests on the cocrystals at 40°C / 75% RH was examined by TGA and XRPD confirmed all cocrystals were unaffected by exposure to humidity.

The cocrystals synthesised in Examples 1 to 13 exhibit an increase in phosphate buffered saline (PBS) 6.8 solubility comparted to the N-1 form of APX and are kinetically stable following dissolution experiments.

Additionally, the cocrystals can be synthesised at scale in water at ambient temperature. Moreover, the coformers used to prepare these cocrystals are generally recognised as safe (GRAS or pharmaceutically approved), low cost and commercially available. Finally, exposure of the cocystals to 40°C and 75% RH for several weeks confirmed that they have acceptable stability for use in drug products without the need for special precautionary storage measures.

### Example 15: Single crystal of APXFUM

A single crystal comprising apixaban (APX) and fumaric acid (FUM) was synthesised in methanol (Honeywell, 99.9 % purity).

10 mg of APXFUM (Example 1) was dissolved in 3 mL of methanol by heating to 50°C in a capped vial. The vial was subsequently uncapped and the mixture was cooled to 35°C which allowed the solvent to evaporate. After three days, needle shaped single crystals suitable for SCXRD were harvested for further evaluation.

The single crystal of APXFUM was mounted on a MiTeGen MicroMounts^{™} precision tool prior to data collection. The data was collected on a Bruker Kappa Apex diffractometer equipped with MoKα radiation (wavelength of λ= 0.7103 Å) with an APEX II CCD detector at 298 ± 2 K. Data collection and data processing were performed with the Bruker APEX III software package. The crystal structure of APXFUM was solved using the Olex2 software package using direct methods and refined against F2 using SHELXL97. Non-hydrogen atoms were refined anisotropically and hydrogen atoms were positioned geometrically. The calculated XRPD pattern was generated using Mercury 2020.1.

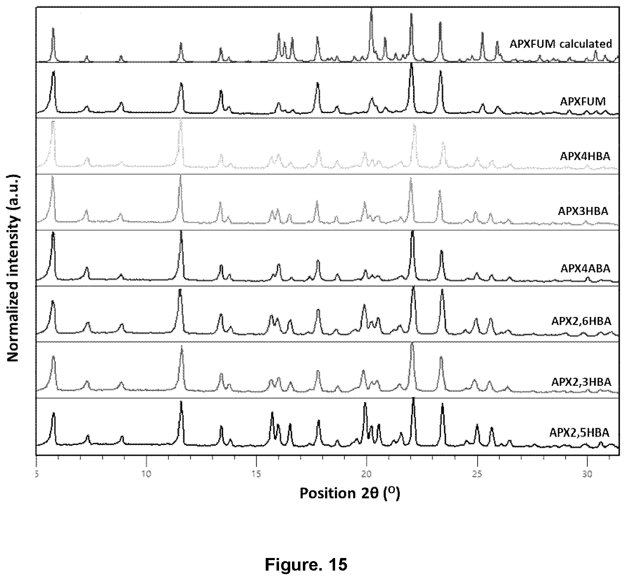

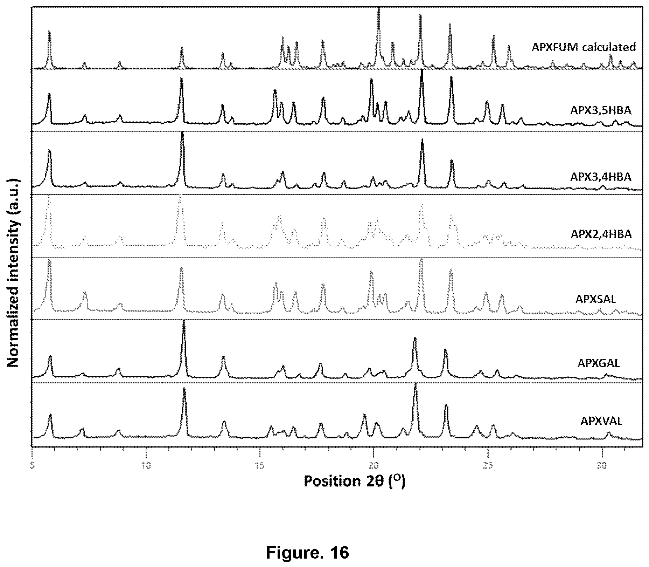

SCXRD confirms that the structure is that of an anhydrous cocrystal. The calculated XRPD pattern from the SCXRD determined structure of Example 15 is in good agreement with the experimental powder patterns of the cocrystals disclosed in Examples 1 to 13 (as shown in Figures 15 and 16).

In summary, the present invention provides cocrystals of apixaban which are kinetically stable and thermodynamically stable and show an increase in solubility at approximately pH 7 compared to apixaban. The cocrystals may be prepared using water as a solvent which is non-toxic and environmentally friendly compared to methods of the prior art which make use of toxic solvents and/or high temperature synthesis methods.

Although a few preferred embodiments have been shown and described, it will be appreciated by those skilled in the art that various changes and modifications might be made without departing from the scope of the invention, as defined in the appended claims.

The optional features set out herein may be used either individually or in combination with each other where appropriate and particularly in the combinations as set out in the accompanying claims. The optional features for each aspect or exemplary embodiment of the invention as set out herein are also to be read as applicable to any other aspect or exemplary embodiments of the invention, where appropriate. In other words, the skilled person reading this specification should consider the optional features for each exemplary embodiment of the invention as interchangeable and combinable between different exemplary embodiments.

Attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, and drawings), and/or all of the steps of any method or process so disclosed, may be combined in any combination, except combinations where at least some of such features and/or steps are mutually exclusive.

Each feature disclosed in this specification (including any accompanying claims, and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, and drawings), or to any novel one, or any novel combination, of the steps of any method or process so disclosed.

## Claims

1. A cocrystal of formula APX·Bx wherein APX is apixaban (1-(4-methoxyphenyl)-7-oxo-6-(4-(2-oxopiperidin-1-yl)phenyl)-4,5,6,7-tetrahydro-1*H*-pyrazolo[3,4-*c*]pyridine-3-carboxamide), B is a pharmaceutically acceptable carboxylic acid selected from fumaric acid or an aromatic carboxylic acid; and x is from 0.1 to 1.0.

2. The cocrystal according to claim 1, wherein the cocrystal is anhydrous.

3. The cocrystal according to any preceding claim, wherein the cocrystal has a X-ray powder diffraction (XRPD) pattern comprising any one or more characteristic peaks at an angle 2θ of 5.7°±0.2°, 7.3°±0.2°, 8.8°±0.2°, 11.5°±0.2°, 20.0°±0.2°, 22.0°±0.2° and 23.3°±0.2° as measured by Cu Kα radiation.

4. The cocrystal according to claim 3, wherein the cocrystal has a X-ray powder diffraction (XRPD) pattern comprising characteristic peaks at an angle 2θ of 5.7°±0.2°, 7.3°±0.2°, 8.8°±0.2°, 11.5°±0.2°, 20.0°±0.2°, 22.0°±0.2° and 23.3°±0.2° as measured by Cu Kα radiation.

5. The cocrystal according to any preceding claim, wherein the aromatic carboxylic acid is a substituted benzoic acid, suitably wherein the benzoic acid is substituted with one or more of C₁-C₄ alkoxy, hydroxy or amino groups.

6. The cocrystal according any preceding claim, wherein the pharmaceutically acceptable carboxylic acid is selected from fumaric acid (FUM), gallic acid (GAL), salicylic acid (SAL), vanillic acid (VAL), 2,3-dihydroxybenzoic acid (2,3HBA), 2,4-dihydroxybenzoic acid (2,4HBA), 2,5-dihydroxybenzoic acid (2,5HBA), 2,6-dihydroxybenzoic acid (2,6HBA), 3,4-dihydroxybenzoic acid (3,4HBA), 3,5-dihydroxybenzoic acid (3,5HBA), 3-hydroxybenzoic acid (3HBA), 4-hydroxybenzoic acid (4HBA) or 4-aminobenzoic acid (4ABA).

7. The cocrystal according to any preceding claim, wherein the cocrystal has a melting point of at least 100°C, preferably from 180 to 230°C.

8. The cocrystal according to any preceding claim, wherein the cocrystal has a higher solubility in phosphate buffered saline compared to apixaban at approximately pH 7 and 37°C.

9. The cocrystal according to any preceding claim, wherein the cocrystal is stable when exposed to a relative humidity of at least 70%.

10. A method of preparing a cocrystal according to any preceding claim, the method comprising:
(a) admixing apixaban and a pharmaceutically acceptable carboxylic acid selected from fumaric acid or an aromatic carboxylic acid in a solvent; and
(b) stirring the mixture obtained in step (a) at room temperature.

11. The method according to claim 10, wherein the solvent is an aqueous solvent, suitably wherein the solvent comprises water.

12. The method according to claim 10 or claim 11, further comprising:
(c) removing the solvent from the mixture obtained in step (b) to obtain the cocrystal.

13. A pharmaceutical composition comprising a cocrystal according to any one of claims 1 to 9 and a pharmaceutically acceptable excipient.

14. The cocrystal according to any one of claims 1 to 9 for use as a medicament (for example wherein the medicament is an anticoagulant).

15. The cocrystal according to any one of claims 1 to 9 for use in the treatment and/or prevention of pulmonary embolism, atrial fibrillation or deep vein thrombosis.
